# EUROPEAN PATENT APPLICATION

(11) **EP 4 645 339 A1**
(43) Date of publication of application: **05.11.2025**
(21) Application number: 23912927.3
(22) Date of filing: 27.12.2023
(51) Int. Cl.: G16H 50/50, G16H 50/30, G06N 3/0464, A61B 5/00, A61B 5/369, A61B 5/318, A61B 5/11

(54) **SEIZURE MANAGEMENT SYSTEM AND METHOD THEREOF**

(30) Priority: 28.12.2022 KR 20220187175
(71) Applicant: SK Biopharmaceuticals Co., Ltd., Seongnam-si, Gyeonggi-do 13494 (KR)
(72) Inventor: PARK, Chan Ho, Seongnam-si, Gyeonggi-do 13494 (KR); KIM, Yongwook, Seongnam-si, Gyeonggi-do 13494 (KR)
(74) Representative: Vossius & Partner Patentanwälte Rechtsanwälte mbB
(86) International application number: PCT/KR2023/021722
(87) International publication number: WO 2024/144253

(57) **Abstract**

A seizure monitoring system according to the present disclosure detects seizure detection and prediction results from bio-signals of the user by using a common seizure Al model that compares similarity with a common seizure template composed of bio-signals collected from a plurality of users, accumulates user data including the bio-signals of the user and seizure detection and prediction results during a first period time, updates the common seizure template and the common seizure Al model using the accumulated user data, generates a personal seizure Al model using the accumulated user data, and determines a model optimized for the user among the updated common seizure Al model or personal seizure Al model.

## Description

### [Technical Field]

This disclosure relates to a seizure monitoring system and method thereof for managing seizures of patient with epilepsy, etc. in real time.

### [Background Art]

An artificial intelligence (Al), which has recently received a lot of attention, is also attracting attention in the field of digital healthcare. There are many fields related to Al, but research in fields related to epilepsy is being actively conducted.

The epilepsy is a disease in which some brain neurons generate excessive electric current for a short period of time, and the main symptom is epileptic seizures that occur in the whole body or part of the body. The excessive electrical currents in brain neurons that cause seizures may be observed through electroencephalography (EEG). Changes in brain waves affect bio-signals such as the electrocardiogram (ECG), ballistocardiogram (BCG), and photoplethysmogram (PPG). Therefore, the epileptic seizures may be detected or predicted based on bio-signals.

Currently, research on epilepsy seizures using artificial intelligence (Al) is focused on seizure prediction research that detects the time of seizure occurrence or predict the time of seizures through the symptoms of precursors and actions according to the seizure prediction.

To predict seizures using AI, Al-based seizure prediction typically involves collecting bio-signals from patients, extracting signal features associated with the symptoms of each patient, and repeatedly learning an Al model using the extracted signal features to implement an Al model optimized for characteristics of each individual patient.

In order to optimize the Al model to suit the characteristics of each individual patient, a certain amount of learning data corresponding to each individual patient must be collected. However, for patients who experience seizures several times a day or more than several times a week, the collection period of the learning data becomes longer, and seizure detection and prediction become impossible during this collection period.

Furthermore, for patients whose seizure frequency is only a few times per month or year, the collection period of the learning data is longer.

There is a need for methods to perform seizure detection and prediction even in situations where seizure data of the individual patient are not sufficiently collected.

### [Disclosure]

### [Technical Problem]

The present disclosure has been made in an effort to provide a seizure monitoring system and method epilepsy patients, which are capable of detecting and predicting individual patient seizures even in situations where seizure data of individual patient is not sufficiently collected.

### [Technical Solution]

According to one embodiment, A seizure monitoring method of a user which is executed by at least one processor is provided. The seizure monitoring method includes: detecting seizure detection and prediction results from bio-signals of the user by using a common seizure Al model that compares similarity with a common seizure template composed of bio-signals collected from a plurality of users; accumulating user data including the bio-signals of the user and seizure detection and prediction results during a first period of time; updating the common seizure template and the common seizure Al model using the accumulated user data; generating a personal seizure Al model using the accumulated user data; and determining a model optimized for the user among the updated common seizure Al model or personal seizure Al model.

The seizure monitoring method may further include updating or replacing the model optimized for the user by using user data additionally accumulated during a second period of time after the first period of time.

The determining may include: evaluating the updated common seizure Al model and the personal seizure Al model using evaluation data selected from the accumulated user data; and selecting the personal seizure Al model as the optimized model for the user if a performance of the personal seizure Al model is better than the updated common seizure Al model by more than or equal to a set performance increase, based on evaluation results.

The seizure monitoring method may further include selecting seizure data and pre-seizure data to be used as learning data from the user data before the updating.

The seizure detection and prediction results include a seizure probability at each time point, and the selecting may include receiving user seizure confirmation information including whether the user has an actual seizure and a seizure time from the user terminal; identifying whether an actual seizure occurred to the user at time points having a seizure probability higher than or equal to a threshold among the bio-signals of the user through the user seizure confirmation information; and determining seizure data by using bio-signals at time points at which an actual seizure is identified to have occurred to the user among the time points having a seizure probability higher than or equal to the threshold value for the bio-signal of the user.

The determining the seizure data may include identifying a seizure type of a bio-signal at each time point having a seizure probability greater than or equal to the threshold; and storing bio-signals of a first seizure type as the seizure data in a seizure template of the first seizure type when a set number or more of bio-signals of the first seizure type are observed among the bio-signals at time points at which the actual seizure is identified to have occurred to the user while having a seizure probability greater than or equal to the threshold.

The determining the seizure data may further include storing the bio-signals at a time point having the first seizure type in a seizure signal candidate group of the first seizure type until the set number or more of bio-signals at a time point having the first seizure type are observed.

The determining the seizure data may further include, if a seizure template of a seizure type identical to a bio-signal at a time point at which the actual seizure is identified to have occurred to the user while having a seizure probability greater than or equal to the threshold exists, storing the bio-signal at the time point as the seizure data in the seizure template.

The selecting may include identifying whether an actual seizure occurred to the user through the user seizure confirmation information at time points having a seizure probability less than the threshold among the bio-signals of the user; and determining the seizure data by using bio-signal at time points at which an actual seizure is identified to have occurred to the user among bio-signals at time points having a seizure probability less than the threshold.

The determining the seizure data may include: identifying a seizure type of a bio-signal at each time point having a seizure probability less than the threshold; storing bio-signals of a second seizure type as an abnormal signal data in an abnormal signal template of the second seizure type, when a set number or more of bio-signals of the second seizure type are observed among the bio-signals at time points at which the actual seizure is identified to have occurred to the user while having a seizure probability less than the threshold; and changing the abnormal signal template of the second seizure type as the seizure data to a seizure template of the second seizure type based on analysis results of the abnormal signal template of the second seizure type.

The determining the seizure data may further include storing the bio-signals at a time point having the second seizure type in an abnormal signal candidate group of the second seizure type until the set number or more of bio-signals at a time point having the second seizure type are observed.

The determining the seizure data may further include, if a seizure template of a seizure type identical to a bio-signal at a time point at which the actual seizure is identified to have occurred to the user while having a seizure probability less than the threshold exists, storing the bio-signal at the time point as the seizure data in the seizure template.

The selecting may include storing the seizure data selected seizure data from the user data in a seizure template for each seizure type, and the updating may include storing the seizure template for each seizure type in the common seizure template.

The seizure monitoring method may further include accumulating data of the plurality of users including bio-signals of plurality of users and seizure detection and prediction results during a third period of time; and optimizing the common seizure Al model by using the data of the plurality of users accumulated during the third period of time.

The optimizing may include: evaluating the common seizure Al model updated using the data of the plurality of users and the common seizure Al model before the update using evaluation data selected from the data of the plurality of users accumulated during the third period of time; and determining one of the updated common seizure Al model and the common seizure Al model before the update as the optimized common seizure Al model based on evaluation results using the data of the plurality of users.

The detecting may include preprocessing the bio-signals of the user; and inputting the preprocessed data into the common seizure Al model.

The preprocessing may include extracting feature data to be used as input for the common seizure Al model.
the bio-signals of the user may include a bio-signal of a new user who does not have the acquired bio-signal or a bio-signal of a new seizure type that is not included in a seizure template of an existing user whose bio-signal has already been input into the common seizure Al model.

The seizure monitoring method may further include detecting seizure detection and prediction results from the bio-signals of the user using the model optimized for the user.

According to another embodiment, a seizure detection and prediction apparatus for seizure monitoring is provided. The seizure detection and prediction apparatus includes: a memory configured to store acquired bio-signals; a learning unit configured to learn a plurality of candidate seizure Al models using learning data selected from the acquired bio-signals, determine one of the plurality of candidate seizure Al models as a common seizure Al model to be used for seizure detection and prediction using evaluation data selected from the acquired bio-signals, and store the common seizure Al model in the memory; and a seizure detector and predictor configured to load the common seizure Al model stored in the memory and output seizure detection and prediction results from the bio-signals of the user using the common seizure Al model.

The seizure detector and predictor may include a bio-signal preprocessor configured to preprocess the bio-signals of the user and extract feature data; a result calculator configured to generate seizure detection and seizure prediction results by using a seizure probability and a pre-seizure probability output from the common seizure Al model that received the feature data as input; and output unit configured to output the seizure detection and seizure prediction results.

The common seizure Al model may include, at least one of a seizure detection model configured to output the seizure probability from the feature data and a seizure prediction model configured to output pre-seizure probability from the feature data.

The common seizure Al model stored in the memory may be configured to update or replace using the seizure detection and seizure prediction results of the plurality of users accumulated during a set period of time.

According to another embodiment, a model optimization apparatus that optimizes a seizure detection and prediction model for seizure detection is provided. The model optimization apparatus includes: a bio-signal accumulator configured to accumulate user data including seizure detection and prediction results detected from bio-signals of a user during a first period of time by using a common seizure Al model learned using learning data selected from acquired bio-signals; a seizure timing selector configured to select bio-signals at seizure times from the user data accumulated during the first period of time; and a personal model generator configured to select learning data and evaluation data from user data using the bio-signals at the seizure times, update the common seizure Al model using the learning data, generate a personal seizure Al model using the learning data, and determine a model optimized for the user among the updated common seizure Al model or the personal seizure Al model.

The personal model generator may be configured to evaluate the updated common seizure Al model and the personal seizure Al model using the evaluation data, and select the personal seizure Al model as the model optimized for the user if a performance of the personal seizure Al model is better than a performance of the updated common seizure Al model based on evaluation results.

The common seizure Al model optimizer may be configured to select learning data and evaluation data from the data of a plurality of users by using bio-signals at seizure times of the plurality of users selected from the user data of the plurality of users accumulated during a second period of time, and optimize the common seizure Al model by using the learning data selected from the data of the plurality of users.

The common seizure Al model optimizer may be configured to update the common seizure Al model using learning data selected from the data of the plurality of users, and evaluate the updated common seizure Al model and the common seizure Al model before the update using evaluation data selected from the data of the plurality of users to determine an optimized common seizure Al model.

The seizure detection and prediction results may include a seizure probability at each time point, bio-signal accumulator may be configured to accumulate user seizure confirmation information including whether the user has an actual seizure and a seizure time, received from the user terminal, as the user data, and seizure timing selector may be configured to determine seizure data by using bio-signals at time points at which an actual seizure is identified to have occurred to the user through user seizure confirmation information, among the time points having a seizure probability higher than or equal to the threshold value for the bio-signals of the user.

The seizure timing selector may be configured to determine the seizure data by using bio-signals at time points at which an actual seizure is identified to have occurred to the user through user seizure confirmation information, among at time points having a seizure probability less than the threshold for the bio-signals of the user.

The common seizure Al model and the personal AI seizure model may include least one of a seizure detection model configured to output a seizure probability and seizure prediction model configured to output a pre-seizure probability, the personal model generator may be configured to set the bio-signals at seizure times as seizure data and set bio-signals at time points other than the seizure time as inter- seizure data, and select them as learning data for learning a seizure detection model, and the personal model generator may be configured to set bio-signals at a predetermined predicted time point prior to the seizure time as pre-seizure data and set bio-signals at time points other than the seizure time and the predicted time point as inter-seizure data, and select them as learning data for learning the seizure prediction model.

### [Advantageous Effects]

According to at least one embodiment, a common seizure Al model that compares similarity with a common seizure template composed of bio-signals collected from a plurality of users who have already acquired is used to detect seizure and seizure prediction results from the bio-signals of a user, thereby monitoring the seizures of the user.

According to at least one embodiment, even if the seizure data of the user is not sufficiently collected, seizure detection and prediction of the user may be possible using the common seizure Al model applicable to the plurality of users.

In addition, according to at least one embodiment, a personalized seizure Al model optimized for the user may be implemented using relatively accurate seizure data of the user according to a time accompanying the actual seizure, and accordingly, highly accurate seizure detection and prediction may be provided to the user.

### [Description of the Drawings]

FIG. 1 is a schematic diagram illustrating a seizure monitoring system according to one embodiment.
FIG. 2 is a block diagram showing the detailed configuration of the seizure detection and prediction apparatus illustrated in Figure 1.
FIG. 3 is a diagram illustrating the common seizure Al model illustrated in FIG. 1.
FIG. 4 is a flowchart showing a learning method of the learning unit illustrated in FIG. 2.
FIG. 5 is a flowchart illustrating a seizure detection and prediction method of the seizure detector and predictor illustrated in FIG. 2.
FIG. 6 is a block diagram showing the detailed configuration of the model optimization apparatus illustrated in FIG. 1.
FIG. 7 is a flowchart illustrating a method for selecting data on the timing of a seizure from the bio-signals of a user whose seizure probability is greater than a threshold value in the seizure timing selector illustrated in FIG. 6.
FIG. 8 is a flowchart illustrating a method for selecting data on the timing of a seizure from the bio-signals of a user whose seizure probability is less than a threshold value in the seizure timing selection unit illustrated in FIG. 6.
FIG. 9 is a diagram illustrating an example of generating a seizure template when the seizure probability is greater than a threshold in the seizure detection and prediction results of the common seizure Al model.
FIG. 10 is a diagram illustrating an example of generating a seizure template when the seizure probability is less than a threshold in the seizure detection and prediction results of the common seizure Al model.
FIG. 11 is a flowchart illustrating a method for generating a personal seizure Al model in the model optimization apparatus illustrated in FIG. 6.
FIG. 12 is a flowchart illustrating a method for optimizing the common seizure Al model in the model optimization apparatus illustrated in FIG. 6.
FIG. 13 is a diagram illustrating a seizure detection and prediction apparatus according to another embodiment.
FIG. 14 is a diagram showing a model optimization apparatus according to another embodiment.

### [Detailed description of the embodiments]

Hereinafter, embodiments of the disclosure will be described in detail with reference to the attached drawings so that a person of ordinary skill in the art may easily implement the disclosure. As those skilled in the art would realize, the described embodiments may be modified in various different ways, all without departing from the spirit or scope of the disclosure. The drawings and description are to be regarded as illustrative in nature and not restrictive. Like reference numerals designate like elements throughout the specification.

Throughout the specification and claims, if a part is referred to "include" a certain element, it may mean that it may further include other elements rather than exclude other elements, unless specifically indicated otherwise.

In addition, throughout the specification and claims, the suffixes "module," "part," and/or "unit" for components are assigned or mixed only for the convenience of writing the specification, and do not in themselves have distinct meanings or roles.

Furthermore, throughout the specification and claims, terms including ordinal numbers, such as first, second, etc., may be used to describe various components, but the components are not limited by the terms. The terms may be used only to distinguish one component from another. For example, without departing from the scope of the present disclosure, a first component could be termed a second component, and similarly, a second component could also be termed a first component.

Throughout the specification and claims, when a component is referred to be "connected" with another component, it includes not only the case where two components are "directly connected" but also the case where two components are "indirectly or non-contactedly connected" with another component interposed therebetween, or the case where two components are "electrically connected." On the other hand, when it is said that a component is "directly connected" to another component, it should be understood that there are no other components in between.

In the flowcharts described with reference to the drawings in this specification, the order of operations may be changed, several operations may be merged, some operations may be divided, and specific operations may not be performed.

Furthermore, in this specification, each of the phrases such as "A or B", "at least one of A and B", "at least one of A or B", "A, B, or C", "at least one of A, B, and C", and "at least one of A, B, or C" may include any one of the items listed together in the corresponding one of the phrases, or all possible combinations thereof.

In addition, in the present disclosure, terms such as "unit," "part" and "module" mean a unit that processes at least one function or operation, which may be implemented by hardware, software, or a combination of hardware and software.

Now, a seizure monitoring system and method according to an embodiment of the present disclosure will be described in detail with reference to the drawings.

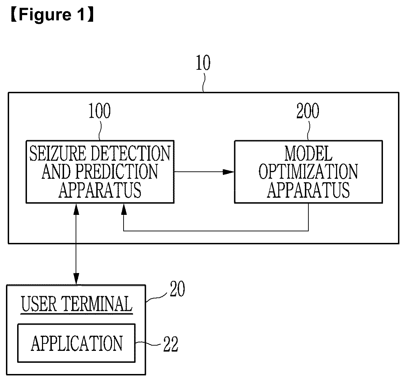

FIG. 1 is a schematic diagram illustrating a seizure monitoring system according to one embodiment.

Referring to FIG. 1, the seizure monitoring system 10 may include a seizure detection and prediction apparatus 100 and a model optimization apparatus 200.

The seizure detection and prediction apparatus 100 trains candidate seizure Al models based on acquired bio-signals, and sets a candidate seizure Al model with the best performance among the trained candidate seizure Al models as a common Al model, and stores the common seizure Al model. The acquired bio-signals may include bio-signals collected from a plurality of users. The acquired bio-signals may include a common seizure template storing bio-signals at the time of seizure of the plurality of users and non-seizure bio-signals between seizures of the plurality of users. The common seizure Al model is not a model optimized for a specific user, but a model that may be universally used by all users for seizure detection and prediction.

The seizure detection and prediction apparatus 100 outputs seizure detection results and seizure prediction results from the newly measured user's bio-signals using a common seizure Al model. The common seizure Al model may output seizure detection results and seizure prediction results from the bio-signals of user by comparing the similarity with common seizure template. The newly measured user's bio-signals may be received from a bio-signal measurement wearable device that worn by the user. The newly measured user's bio-signals may include bio-signals of a new user for whom no bio-signals have been acquired, or new types of bio-signals that are not included in the seizure templates of existing users.

The model optimization apparatus 200 collects and accumulates bio-signals measured from each user from the seizure detection and prediction apparatus 100, and selects learning data to be used for implementing a model optimized for each user from the accumulated bio-signals of each user.

The model optimization apparatus 200 trains a personal seizure Al model and a common seizure Al model using the learning data of each user, thereby implementing the personal seizure Al model and updating the common seizure Al model. The model optimization apparatus 200 may evaluate the personal seizure Al model and the updated common seizure Al model using evaluation data, which is some of the learning data of each user, and determine the personal seizure Al model or the updated common seizure Al model as a model to be used for the user based on evaluation results.

In addition, the model optimization apparatus 200 may update the common seizure Al model using learning data selected by using bio-signals of the plurality of users accumulated at a set period of time. The model optimization apparatus 200 may evaluate the updated common seizure Al model and the common seizure Al model before the update using evaluation data, which is part of the learning data, and determine the updated common seizure Al model or the common seizure Al model before the update as the optimized common seizure Al model based on the evaluation results.

The seizure detection and prediction apparatus 100 may replace or update the common seizure Al model with a common seizure Al model optimized by the model optimization apparatus 200.

The user terminal 20 may request seizure detection and prediction from the seizure detection and prediction apparatus 100 and receive seizure detection and prediction results from the seizure detection and prediction apparatus 100.

The user terminal 20 may have an application 22 installed that may request seizure detection and prediction and receive and display seizure detection and prediction results from the seizure detection and prediction apparatus 100. That is, a user may request seizure detection and prediction by executing the application 22 and receive seizure detection and prediction results through the application 22.

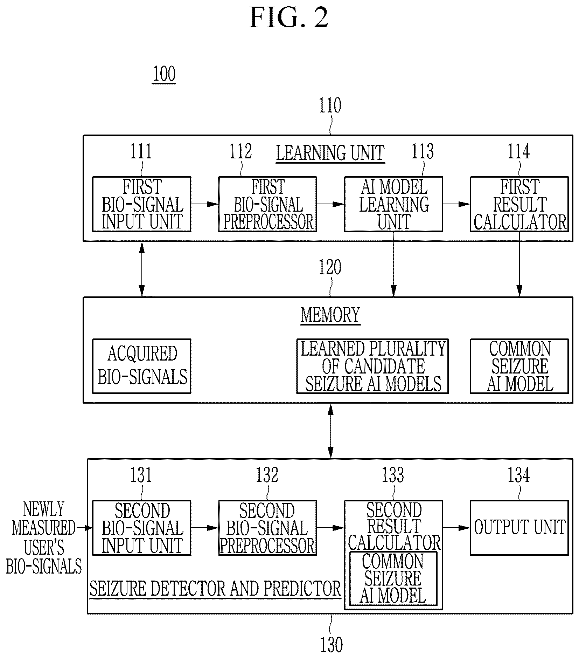

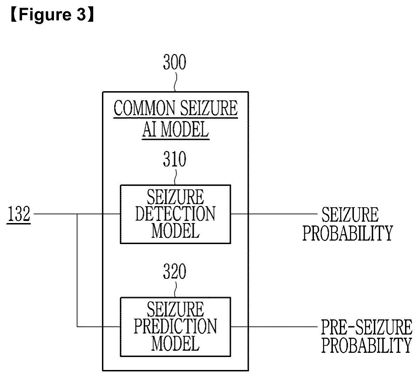

FIG. 2 is a block diagram showing the detailed configuration of the seizure detection and prediction apparatus illustrated in Figure 1, and FIG. 3 is a diagram illustrating the common seizure Al model illustrated in FIG. 1.

Referring to FIG. 2, the seizure detection and prediction apparatus 100 may include a learning unit 110, a memory 120, and a seizure detector and predictor 130.

The learning unit 110 learns a plurality of candidate seizure Al models using the acquired bio-signals, and determines a candidate seizure Al model with the best performance among the plurality of candidate seizure Al models.

The learning unit 110 may include a first bio-signal input unit 111, a first bio-signal preprocessor 112, an Al model learning unit 113, and a first result calculator 114.

The first bio-signal input unit 111 reads the acquired bio-signals from the memory 120. The acquired bio-signals are digital multimodal bio-signals, which may include brain waves of the head, electrocardiograms measured from the head or chest, acceleration and angular velocity reflecting the movement of the head or body, etc., measured by bio-signal measurement wearable device worn by each of a plurality of users, and at least one type of signal and a combination of two or more types of signals may be used. In one embodiment, it may be a combination of brain waves measured from the head and electrocardiograms measured from the head.

The first bio-signal preprocessor 112 performs preprocessing on the acquired bio-signals inputted through the first bio-signal input unit 111.

The preprocessing of acquired bio-signals may include removing noise signals resulting from movement, eye blinking, and chin movement. The noise signal removal method may include a method of extracting noise signals and removing the noise signals from the bio-signals by using template matching, or a method of removing noise components by decomposing the bio-signals by using a method such as independent component analysis (ICA) or empirical mode decomposition (EMD).

The preprocessing of the acquired bio-signals may include a process of limiting a frequency band of the multimodal bio-signals using a band-pass filter. For example, the frequency band of each type of bio-signals may be limited according to the characteristics of each type of bio-signals, such as extracting signals from 0.5 Hz to 50 Hz in brain waves or signals from 10 Hz to 25 Hz in electrocardiograms.

The preprocessing of the acquired bio-signals may include a process of dividing the bio-signals using moving window of a specific length (e.g., 10 seconds). The preprocessing of the acquired bio-signals may include a process of dividing the bio-signals by moving the moving window by a specific length (e.g., 1 second).

In addition, the preprocessing of the acquired bio-signals may include processes such as calculating time and frequency features for each divided data and calculating coherence features. Time and frequency feature calculations may use a short-time Fourier transform (STFT) or wavelet transform method.

The feature data preprocessed by the first bio-signal preprocessor 112 is transmitted to the Al model learning unit 113.

The Al model learning unit 113 learns a plurality of candidate seizure Al models using feature data of bio-signals preprocessed by the first bio-signal preprocessor 112. The plurality of candidate seizure Al models may include one-dimensional convolutional neural network models, two-dimensional convolutional neural network models, and combinations of these models.

The plurality of candidate seizure Al models may be learned using feature data of multimodal bio-signals of the plurality of users. Each candidate seizure Al model may include a seizure detection model for seizure detection and a seizure prediction model for seizure prediction. The feature data of the bio-signals input to the seizure detection model may be feature data of the bio-signals at the time of the seizure. The feature data of bio-signals input into the seizure prediction model may be the feature data of bio-signals within several minutes or several hours before the seizure.

The seizure detection model detects the timing of a seizure. The seizure detection model distinguishes between signals at the time of the seizure (or feature data extracted from signals at the time of the seizure) and non-seizure signals (or feature data extracted from non-seizure signals), and may use seizure-time signals and non-seizure signals as learning data.

The seizure prediction model predicts the timing of a seizure. The seizure prediction model distinguishes between signals at the predicted time (or feature data extracted from signals at the predicted time) within several minutes or several hours before the time of the seizure, excluding the time of the seizure, and signals other than this time (or feature data extracted from signals outside this time), and may use signals at the predicted time and signals at times other than the predicted time and the seizure time as learning data.

The learning data used in the seizure detection model and seizure prediction model may be the remaining data excluding the evaluation data for model evaluation from the entire learning data. For example, in the entire learning data, approximately 1/10 may be used as evaluation data, and the remaining data may be used as learning data for learning.

The learning data may be adjusted by making the size of correct answer data groups and incorrect answer data groups equal or in a specific ratio based on match the size of the smaller data group among the correct answer data group including signals at the time of the seizure and the time of the prediction and the incorrect answer data group including signals at times other than the time of the seizure and the time of the prediction.

The plurality of candidate seizure Al models learned by the Al model learning unit 113 are stored in the memory 120, and the plurality of candidate seizure Al models learned stored in the memory unit 120 are used in the first result calculator 114.

The first result calculator 114 uses feature data used as evaluation data among feature data of previously acquired bio-signals as inputs for the learned plurality of candidate seizure Al models, and determines one candidate seizure Al model to be used as the common seizure Al model from the seizure detection results of the learned plurality of candidate seizure Al models. The first result calculator 114 calculates the performance of the learned plurality of candidate seizure Al models using a method such as detection accuracy or area under receiver operating characteristic (ROC) curve from the seizure detection results of the learned plurality of candidate seizure Al models, and determines the candidate seizure Al model with the best performance as the common seizure Al model.

The evaluation data may be organized by person unit. In addition, the evaluation data may be configured in a way that may represent the acquired bio-signals. For example, if the locations of measurement electrodes of the evaluation data are different or there is no data for a specific electrode, the evaluation data cannot be considered to represent the acquired bio-signals. When the evaluation data is configured, problems may arise if only the bio-signals of individuals with seizure types different from the acquired bio-signals are selected. Therefore, the evaluation data may be configured in a way that may represent the acquired bio-signals, and the methods for representing the acquired bio-signals may include a method of configuring the evaluation data several times and averaging the results, or a method of analyzing the data by increasing the number of people included in the evaluation data to a statistically significant number or more. In addition, when the number of evaluation data is small, K-fold cross-validation or leave-one-out cross-validation (LOOCV) may be used.

The memory 120 may store bio-signals of the plurality of users, the plurality of candidate seizure Al models, the learned plurality of candidate seizure Al models, and the common seizure Al model determined from the learned plurality of candidate seizure Al models. The memory 120 may store the common seizure templates and non-seizure bio-signals between seizures of the plurality of users as previously acquired bio-signals of the plurality of users.

The seizure detector and predictor 130 performs seizure detection and prediction from the newly measured user's bio-signals using the common seizure Al model determined by the learning unit 110.

The seizure detector and predictor 130 may include a second bio-signal input unit 131, a second bio-signal preprocessor 132, a second result calculator 133, and an output unit 134.

The second bio-signal input unit 131 receives the newly measured user's bio-signals as digital signals from a multimodal bio-signal measurement device or a server connected to the multimodal bio-signal measurement device. The second bio-signal input unit 131 stores the bio-signals of the user in the memory 120. The newly measured user's bio-signals may include brain waves of the head, electrocardiograms measured from the head or chest, acceleration and angular velocity reflecting the movement of the head or body, etc., measured by bio-signal measurement wearable device worn by each of a plurality of users, and at least one type of signal and a combination of two or more types of signals may be used. In one embodiment, it may be a combination of brain waves measured from the head and electrocardiograms measured from the head.

The second bio-signal preprocessor 132 performs preprocessing on the newly measured user's bio-signals inputted through the second bio-signal input unit 131. The second bio-signal preprocessor 132 may perform preprocessing in the same manner as the first bio-signal preprocessor 112. In addition, the second bio-signal preprocessor 132 may determine whether the bio-signals has been measured properly. To determine whether the bio-signals has been measured properly, the second bio-signal preprocessor 132 may receive impedance measurement values of electrodes from a multimodal bio-signal measurement device and may determine that the bio-signals are unreliable if the impedance measurement values of the electrodes are greater than or equal to a threshold value, and consider the bio-signals as unusable signals.

The feature data preprocessed by the second bio-signal preprocessor 132 is transmitted to the second result calculator 133.

The second result calculator 133 loads the common seizure Al model stored in the memory 120 and may provide seizure detection and seizure prediction results from the feature data of the newly measured user's bio-signals, using the common seizure Al model. In contrast, the second result calculator 133 may provide seizure detection and seizure prediction results from the user's bio-signals input through the second bio-signal input unit 131 without preprocessing the newly measured user's bio-signals. Below, it is assumed and explained that the preprocessed feature data is used as input data for seizure detection and seizure prediction.

As illustrated in FIG. 3, the common seizure Al model 300 may include a seizure detection model 310 and a seizure prediction model 320. The seizure detection model 310 and the seizure prediction model 320 may be independent of each other. Therefore, an independent seizure detection apparatus may be implemented by the second bio-signal input unit 131, the second bio-signal preprocessor 132, the seizure detection model 310, and the output unit 134. In addition, an independent seizure prediction apparatus may be implemented by the second bio-signal input unit 131, the second bio-signal preprocessor 132, the seizure prediction model 320, and the output unit 134.

The seizure detection model 310 may receive the feature data of the newly measured user's bio-signals as input and output whether it is a seizure or inter-seizure. The seizure detection model 310 may output whether it is a seizure or inter-seizure as a seizure probability.

The seizure prediction model 320 may receive as input the feature data of the newly measured user's bio-signals and output whether it is pre-seizure or inter-seizure. The seizure prediction model 320 may output whether it is pre-seizure or inter-seizure as a pre-seizure probability.

The seizure detection model 310 and the seizure prediction model 320 may output artifacts or disconnected as output results.

Again, referring to FIG. 2, the second result calculator 133 may post-process the seizure probability and pre-seizure probability output from the seizure detection model 310 and the seizure prediction model 320, respectively, and generate a seizure detection result and a seizure prediction result.

The second result calculator 133 may classify the seizure probability as the seizure if the seizure probability is greater than a reference value, and may generate the seizure detection result based on the post-processing result that combines the seizure probability for several seconds or minutes. The post-processing method that may be used a method that calculates an average of the probability results over several seconds. At this time, if the average probability is greater than or equal to 50%, it may be determined as a seizure and a seizure detection result may be generated. The post-processing method may be used a method that is monitor whether the seizure detection result with a probability of 90% or higher occurs more than 5 times during 10 seconds, assuming that the seizure detection result is generated every second. At this time, if the seizure detection result with a probability of 90% or higher occurs more than 5 times during 10 seconds, it may be determined as the seizure. The post-processing method may be used a method that is monitor whether the probability greater than or equal to the reference value occurs continuously a set number of times. If the probability greater than or equal to the reference value occurs consecutively the set number of times, it may be determined as the seizure. In addition, various post-processing methods may be used.

The second result calculator 133 may classify the pre-seizure probability as pre-seizure if the pre-seizure probability is greater than or equal to a reference value, and may generate a seizure prediction result based on the post-processing result that combines the pre-seizure probability for several seconds or minutes. The post-processing method may be the same as the method mentioned above.

The output unit 134 outputs the seizure detection result and seizure prediction result output from the second result calculator 133. The output unit 134 may include a display device or a printer.

In this way, the seizure detector and predictor 130 may output seizure detection and prediction results from newly measured user's bio-signals using the common seizure Al model learned from acquired bio-signals. Therefore, even if there is no accumulated bio-signals of the user, seizure detection and prediction of the user may be performed.

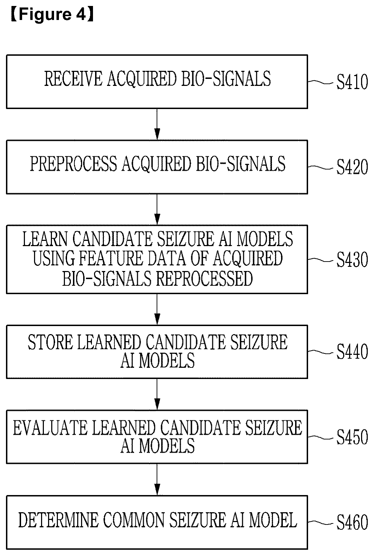

FIG. 4 is a flowchart showing a learning method of the learning unit illustrated in FIG. 2.

Referring to Fig. 4, the learning unit 110 may receive acquired bio-signals through the first bio-signal input unit 111 (S410).

The learning unit 110 performs preprocessing on the acquired bio-signals through the first bio-signal preprocessor 112 (S420).

The learning unit 110 learns a plurality of candidate seizure Al models using feature data of the previously acquired bio-signals preprocessed by the first bio-signal preprocessor 112, through the Al model learning unit 113 (S430).

The learning unit 110 stores the plurality of candidate seizure Al models learned through the Al model learning unit 113 in the memory 120 (S440).

The learning unit 110 evaluates the plurality of candidate seizure Al models learned using feature data used as evaluation data among the feature data of the acquired bio-signals, through the first result calculator 114 (S450).

The learning unit 110 determines the candidate seizure Al model with the best performance as the common seizure Al model using the results of evaluating the plurality of candidate seizure Al models (S460).

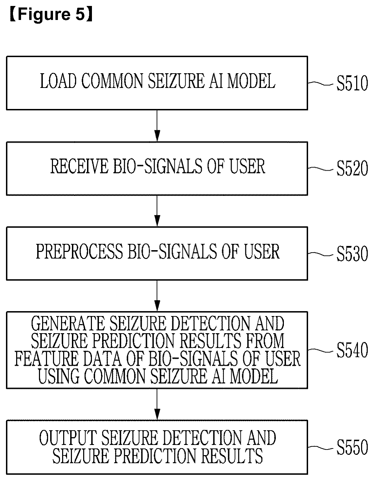

FIG. 5 is a flowchart illustrating a seizure detection and prediction method of the seizure detector and predictor illustrated in FIG. 2.

Referring to Fig. 5, the seizure detector and predictor 130 loads a common seizure Al model from the memory 120 (S510).

The seizure detector and predictor 130 receives bio-signals of a user through the second bio-signal input unit 131 (S520).

The seizure detector and predictor 130 performs preprocessing on the bio-signals of the user inputted through the second bio-signal input unit 131, through the second bio-signal preprocessor 132 (S530).

The seizure detector and predictor 130 generates seizure detection and seizure prediction results from the feature data of the bio-signals of the user, which is preprocessed by the second bio-signal preprocessor 132, using the common seizure Al model, through the second result calculator 133 (S540). The feature data of the bio-signals of the user, which is preprocessed by the second bio-signal preprocessor 132, may be input into the common seizure Al model, and a seizure probability may be output from a seizure detection model of the common seizure Al model, and a pre-seizure probability may be output from a seizure prediction model of the common seizure Al model. The second result calculator 133 may post-process the seizure probability output from the seizure detection model and generate a seizure detection result. The second result calculator 133 may post-process the pre-seizure probability output from the seizure prediction model and generate a seizure prediction result.

The seizure detector and predictor 130 outputs the seizure detection and seizure prediction results to the user through the output unit 134 (S550).

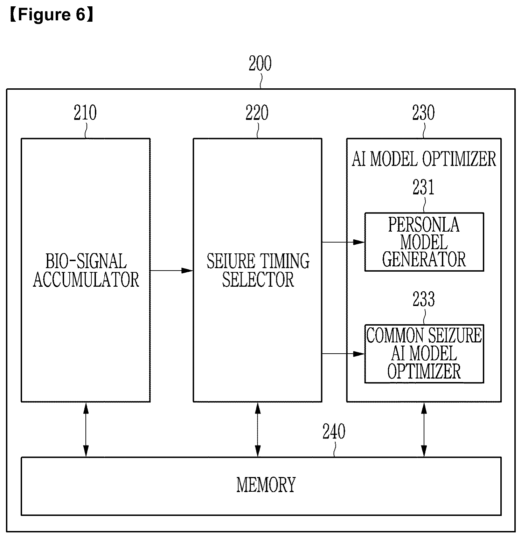

FIG. 6 is a block diagram showing the detailed configuration of the model optimization apparatus illustrated in FIG. 1.

Referring to FIG. 6, the model optimization apparatus 200 may include a bio-signal accumulator 210, a seizure timing selector 220, an Al model optimizer 230, and a memory 240.

The bio-signal accumulator 210 reads the bio-signals of the user inputted through the second bio-signal input unit 131 from the memory 120, and stores the seizure detection and seizure prediction results of the second result calculator 133 for the bio-signals of the user together with the bio-signals of the user in the memory 240. In this way, the bio-signal accumulator 210 stores the bio-signals of each user and the seizure detection and seizure prediction results for the bio-signals of each user in the memory 240. The seizure detection result may include seizure probability and whether or not a seizure has occurred.

The seizure prediction results may include pre-seizure probability and whether or not a pre-seizure has occurred.

The bio-signal accumulator 210 may receive information on whether the user has a seizure through a user terminal or a guardian terminal. When the bio-signal accumulator 210 stores the bio-signals of the user and the seizure detection and seizure prediction results, together with the information on whether the user has a seizure. The information on whether the user has a seizure may include whether an actual seizure occurred and the time of the seizure. The information on whether the user has a seizure may further include at least one of seizure duration, seizure end time, and seizure type.

The information on whether the user has a seizure may be to confirm whether the user had a seizure at the same time as the seizure was determined.

The user terminal or guardian terminal may record the time when a seizure occurs to the user and transmit the seizure time to the model optimization apparatus 200.

The whether or not a seizure has occurred detected by the common seizure Al model 300 may not match actual seizures. The seizure time selector 220 selects data with a high probability of actually being a seizure among the bio-signals of the user accumulated in the bio-signal accumulator 210 as the bio-signal at the seizure time, and stores the selected bio-signals at the seizure time in the memory 240, so that the data at the time at which the actual seizure occurred may be used to optimize the common seizure Al model 300 by the Al model optimizer 230. In addition, when the bio-signals of the seizure time is selected, the seizure time selector 220 selects bio-signals within several minutes or several hours before the same seizure time in the bio-signal of the user to which the selected bio-signals of the seizure time is applied as bio-signals of a predicted time, and stores the bio-signals of the predicted time in the memory 240.

The Al model optimizer 230 may include a personal model generator 231 and a common seizure Al model optimizer 233.

The personal model generator 231 learns the common seizure Al model using the seizure data and pre-seizure data of the user as learning data, and updates the common seizure Al model to be optimized for the user. In addition, the personal model generator 231 learns a separately produced personal seizure Al model using the seizure data and pre-seizure data of the user as learning data, thereby generating a personal seizure Al model for the user.

The personal model generator 231 sets bio-signals at the seizure time selected by the seizure time selector 220 among the bio-signals of the user accumulated in the bio-signal accumulator 210 as seizure data, and sets the bio-signals at the time not selected as the seizure time as inter-seizure data. Next, the personal model generator 231 performs preprocessing on the seizure data and inter-seizure data, and then uses the preprocessed seizure data and inter-seizure data as learning data, and learns the seizure detection model 310 of the common seizure Al model 300 and the seizure detection model of the personal seizure Al model, respectively, using the same learning method as the Al model learning unit 113. At this time, the same preprocessing method as the first bio-signal preprocessor 112 may be used for preprocessing.

The personal model generator 231 sets bio-signals at the predicted time selected by the seizure time selector 220 among the bio-signals of the user accumulated in the bio-signal accumulator 210 as pre-seizure data, and sets data at times other than the predicted time and the seizure time as inter-seizure data. Next, the personal model generator 231 performs preprocessing on the pre-seizure data and inter-seizure data, and then uses the preprocessed pre-seizure data and inter-seizure data as learning data, and learns the seizure prediction model 320 of the common seizure Al model 300 and the seizure prediction model of the personal seizure Al model, respectively, using the same learning method as the Al model learning unit 113. At this time, the same preprocessing method as the first bio-signal preprocessor 112 may be used for preprocessing.

The personal model generator 231 evaluates the learned common seizure Al model and personal seizure Al model using evaluation data, which is some of the selected learning data, and replaces a model to be used for the user from the common seizure Al model to the personal seizure Al model if the performance of the personal seizure Al model is better than the common seizure Al model by more than or equal to the set performance increase.

Next, whenever additional seizure data of the user with an interval longer than a certain period of time is selected, the personal seizure Al model may be updated or replaced using the additional seizure data.

For example, after the initial 30 or more seizure data, whenever 10 additional seizure data are selected with an interval longer than a certain period of time (e.g., 3 hours), the personal model generator 231 may update or replace the personal seizure Al model using the 10 additional seizure data.

The minimum number of seizure times required to generate a new personal seizure Al model may be set to any arbitrary number greater than 30, and the number of seizure times required to generate the new personal seizure Al model and the performance increase required to replace the model may be changed depending on the seizure type of the user or number of seizures per year.

The model optimized for the user is stored in the memory 240, and a personal seizure Al model with low performance may be removed from the memory 240.

The common seizure Al model optimizer 233 uses the preprocessed seizure data and inter-seizure data of the plurality of users selected by the seizure time selector 220 as learning data and learns the seizure detection model 310 of the common seizure Al model 300 using the same learning method as the Al model learning unit 113, thereby optimizing the seizure detection model 310 of the common seizure Al model 300. In addition, the common seizure Al model optimizer 233 uses the preprocessed pre-seizure data and inter-seizure data of the plurality of users selected by the seizure time selector 220 as learning data and learns the seizure prediction model 320 of the common seizure Al model 300 using the same learning method as the Al model learning unit 113, thereby optimizing the seizure prediction model 320 of the common seizure Al model 300.

The common seizure Al model optimizer 233 applies learning data for the plurality of users selected from the accumulated bio-signals of the plurality of users at set intervals (e.g., once per quarter) to the seizure detection model 310 and the seizure prediction model 320 of the common seizure Al model 300 and optimizes seizure detection model 310 and the seizure prediction model 320 in a way that optimizes the parameters of the seizure detection model 310 and the seizure prediction model 320 of the common seizure Al model 300.

The common seizure Al model optimizer 233 evaluates the optimized common seizure Al model and a previous common seizure Al model using evaluation data, which is some of the learning data for the plurality of users, and if the performance of the optimized common seizure Al model is better than the previous common seizure Al model by more than a set performance increase, the seizure detection model 310 and the seizure prediction model 320 of the optimized common seizure Al model 300 are stored in the memory 240, the common seizure Al model with low performance or generated a certain period of time ago may be removed from the memory 240.

The seizure detection model 310 and seizure prediction model 320 of the optimized common seizure Al model 300 stored in the memory 240 are transmitted to the seizure detection and prediction apparatus 100.

Meanwhile, the memory 240 may be identical to the memory 120 illustrated in FIG. 2.

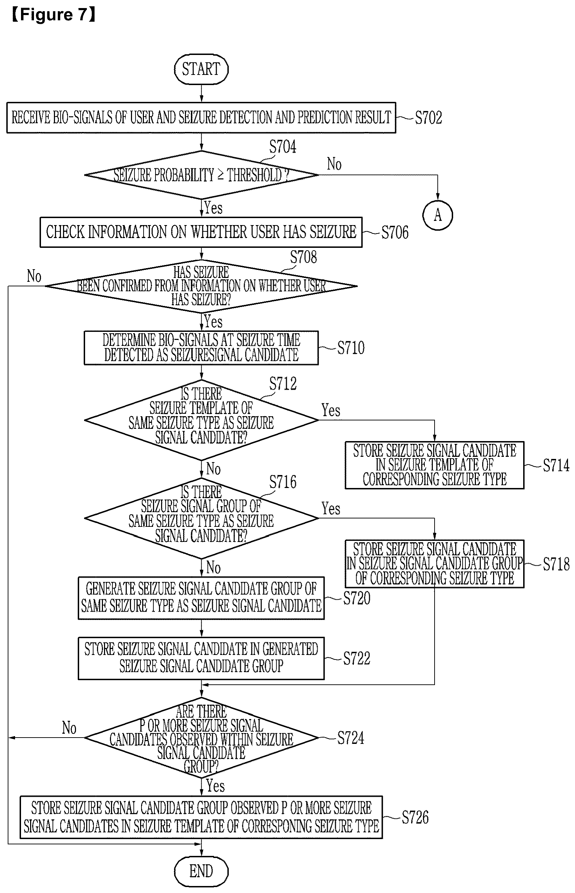

FIG. 7 is a flowchart illustrating a method for selecting data on the timing of a seizure from the bio-signals of a user whose seizure probability is greater than a threshold value in the seizure timing selector illustrated in FIG. 6.

Referring to FIG. 7, the seizure timing selector 220 receives the bio-signals of the user and the seizure detection and seizure prediction results for the bio-signals of the user from the bio-signal accumulator 210 (S702).

The seizure timing selector 220 checks whether the seizure probability is greater than or equal to a threshold value based on the seizure detection and seizure prediction results for the bio-signals of the user (S704).

The seizure timing selector 220 checks information on whether the user has a seizure, reported from a user terminal or guardian terminal, if the seizure probability is greater than or equal to a threshold value (S706).

If the information on whether the user has a seizure is confirmed as a seizure (S708), the seizure time selector 220 may determine the bio-signals at the seizure time detected from the common seizure Al model as a seizure signal candidate (S710).

If a seizure is detected by the common seizure Al model and it is confirmed that the seizure occurred at the same time as the seizure time detected as a seizure by the common seizure Al model, through the information on whether the user has a seizure, it is highly likely that the seizure actually occurred at the seizure time. However, if the detection result of the common seizure Al model and the result confirmed through the information on whether the user has a seizure are different, and the bio-signals at the seizure time are used to optimize the common seizure Al model, it may be a factor that reduces the performance of the model. Operations to complement this are described below.

When the seizure signal candidate is determined, the seizure timing selector 220 checks whether there is a seizure template that includes seizure signals of the same seizure type as the seizure signal candidate (S712). The seizure timing selector 220 may check a similarity between the seizure signal candidate and each seizure template to determine whether there is a seizure template that includes seizure signals of the same seizure type as the seizure signal candidate. A similarity algorithm may be used to check the similarity, and if the similarity output through the similarity algorithm is greater than or equal to a set reference (e.g., 95%), the seizure timing selector 220 may be determined that it is the same seizure type. The similarity algorithm may use techniques such as correlation analysis, dynamic time warping, and canonical correlation analysis, for example.

The seizure timing selector 220 stores the seizure signal candidate in the seizure template if there is a seizure template that includes seizure signals of the same seizure type as the seizure signal candidate (S714).

Meanwhile, if there is no seizure template including seizure signals of the same seizure type as the seizure signal candidate, the seizure timing selector 220 checks whether there is a seizure signal candidate group of the same seizure type as the seizure signal candidate (S716). The seizure timing selector 220 may check a similarity between seizure signal candidates and each seizure signal candidate group to check whether there is a seizure signal candidate group of the same seizure type as the seizure signal candidate.

If there is a seizure signal candidate group of the same seizure type as the seizure signal candidate, the seizure timing selector 220 stores the seizure signal candidate in the seizure signal candidate group of the same seizure type as the seizure signal candidate (S718).

Meanwhile, if there is no seizure signal candidate group of the same seizure type as the seizure signal candidate, the seizure timing selector 220 may generate a seizure signal candidate group of the corresponding seizure type (S720) and store the seizure signal candidate in the generated seizure signal candidate group (S722).

The seizure timing selector 220 checks whether p (e.g., 5) or more seizure signal candidates are observed within the seizure signal candidate group (S724).

If p or more seizure signal candidates of the same seizure type are observed p or more times within the seizure signal candidate group, the seizure timing selector 220 determine all seizure signal candidates of the same seizure type observed p or more times as seizure signals, and may store the seizure signal candidates of the same seizure type as a seizure template of the corresponding seizure type (S726).

The seizure timing selector 220 may store seizure template of each seizure type and seizure signal candidate group of each seizure type in the memory 240. The seizure template of each seizure type may be used as learning data in the Al model optimizer 230. In addition, the seizure template of each seizure type used as learning data may be stored in a common seizure template.

As in step S704, when using the seizure probability and threshold, if only the bio-signals of the user corresponding to the seizure time when the seizure probability is greater than or equal to the threshold among the seizure times detected by the common seizure Al model are used for learning, an overfitting problem of the common seizure Al model may occur. To solve this overfitting problem, the seizure timing selector 220 checks information on whether the user has a seizure when the seizure probability is less than the threshold, and then uses the corresponding bio-signals for learning.

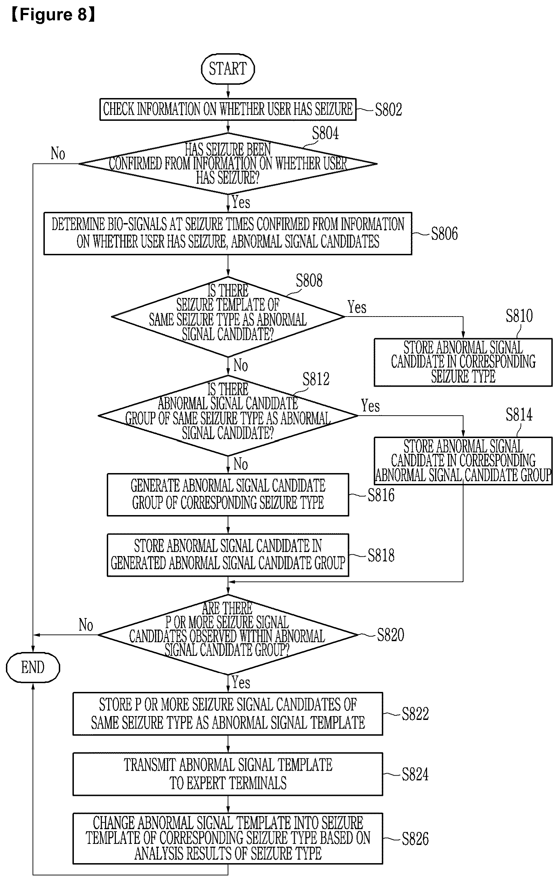

FIG. 8 is a flowchart illustrating a method for selecting data on the timing of a seizure from the bio-signals of a user whose seizure probability is less than a threshold value in the seizure timing selection unit illustrated in FIG. 6.

Referring to FIG. 8, if the seizure probability is less than a threshold, the seizure timing selector 220 information on whether the user has a seizure, reported from a user terminal or guardian terminal (S802).

If the information on whether the user has a seizure is confirmed as a seizure (S804), the seizure timing selector 220 may determine bio-signals at the seizure time confirmed from the information on whether the user has a seizure, although the seizure probability is less than the threshold, as abnormal signal candidates (S806). For example, if the seizure probability is 99%, it is detected as a seizure by the seizure detection model of the common seizure Al model, but since the seizure probability is less than the threshold (e.g., 99.9%), it cannot be selected as learning data through the process of FIG. 7. However, since this may be the case when the learning of the seizure detection model is incomplete due to a lack of learning data of the same type as the corresponding bio-signal, if a seizure is detected as a seizure by the seizure detection model of the common seizure Al model but the seizure probability is below the threshold, if it is confirmed that a seizure occurred at the same time as the seizure time detected as a seizure by the common seizure Al model based on whether the user confirmed a seizure, the bio-signal at that time can be selected as a candidate for an abnormal signal. In addition, in the case where the seizure probability is 47%, the inter-seizure was detected by the seizure detection model of the common seizure Al model, but it may be confirmed that the seizure occurred at the same time as the time detected as inter-seizure by the common seizure Al model based on information on whether the user has a seizure. This may be the case when bio-signals including a seizure waveform that the seizure detection model of the common seizure Al model has not learned is input. Therefore, if a seizure is confirmed to have occurred at the same time based on information on whether the user has a seizure, even though the seizure detection model of the common seizure Al model did not detect the seizure, the bio-signals at the corresponding time may also be determined as an abnormal signal candidate.

When the abnormal signal candidate is determined, the seizure timing selector 220 checks whether there is a seizure template that includes seizure signals of the same seizure type as the abnormal signal candidate (S808). The seizure timing selector 220 may check a similarity between the abnormal signal candidate and each seizure template to determine whether there is a seizure template that includes seizure signals of the same seizure type as the abnormal signal candidate.

If there is a seizure template that includes seizure signals of the same seizure type as the abnormal signal candidate, the seizure timing selector 220 considers the abnormal signal candidate as a seizure signal and stores the abnormal signal candidate to the seizure template (S810).

Meanwhile, if there is no seizure template that includes seizure signals of the same seizure type as the abnormal signal candidate, the seizure timing selector 220 checks whether there is an abnormal signal group of the same seizure type as the abnormal signal candidate (S812). The seizure timing selector 220 may check a similarity between the abnormal signal candidate and each abnormal signal candidate group to determine whether there is an abnormal signal group of the same seizure type as the abnormal signal candidate.

If there is an abnormal signal group of the same seizure type as the abnormal signal candidate, the seizure timing selector 220 stores the abnormal signal candidate in the abnormal signal candidate group of the same seizure type as the abnormal signal candidate (S814).

Meanwhile, if there is no abnormal signal candidate group of the same seizure type as the abnormal signal candidate, the seizure timing selector 220 may generate an abnormal signal candidate group of the corresponding seizure type (S816) and store the abnormal signal candidate in the generated abnormal signal candidate group (S818).

The seizure timing selector 220 checks whether q (e.g., 5) or more abnormal signal candidates are observed within the abnormal signal candidate group (S820).

If q or more abnormal signal candidates of the same seizure type are observed within the abnormal signal candidate group, the seizure timing selector 220 stores q or more abnormal signal candidates of the same seizure type as an abnormal signal template (S822), and may transmit the abnormal signal template to a plurality of expert terminals (S824).

The seizure timing selector 220 may receive analysis results of the abnormal signal template from the plurality of expert terminals. The seizure timing selector 220 may change the abnormal signal template to a seizure template of the corresponding seizure type based on the analysis results of the abnormal signal template (S826). If the abnormal signal template is determined to be a seizure signal from more than a set reference value (e.g., a majority) of expert terminals among the plurality expert terminals, the seizure timing selector 220 determines that abnormal signal candidates within the corresponding abnormal signal template as seizure signals and may change the abnormal signal template to a seizure template of the corresponding seizure type.

The seizure timing selector 220 may store a seizure template of each seizure type and an abnormal signal candidate of each seizure type in the memory 240. The seizure template of each seizure type may be used as learning data in the Al model optimizer 230. In addition, the seizure template of each seizure type used as learning data may be stored in the common seizure template.

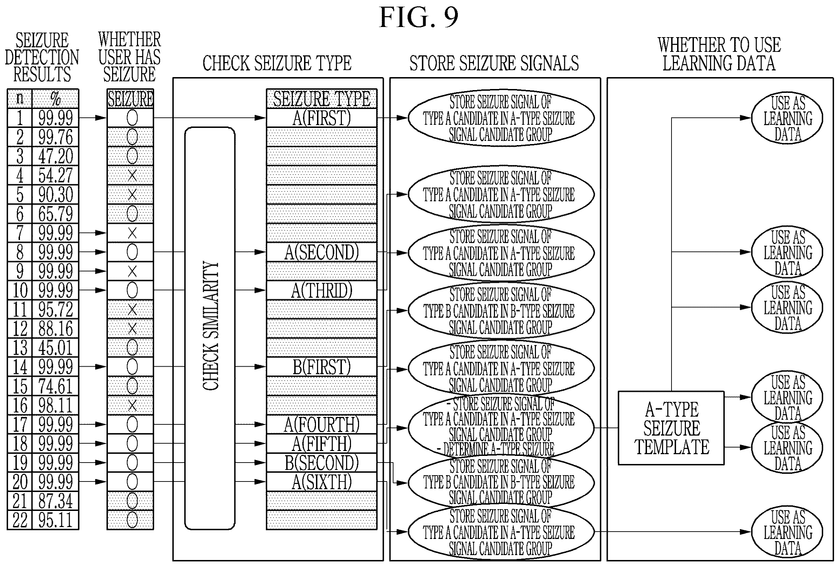

FIG. 9 is a diagram illustrating an example of generating a seizure template when the seizure probability is greater than a threshold in the seizure detection and prediction results of the common seizure Al model. Here, the threshold was assumed to be 99.9%, and 22 seizure detection and prediction results in total, including 20 seizure probabilities detected as seizure and the 2 seizure probabilities detected between as inter-seizure, were shown.

Referring to FIGS. 7 and 9, in step S704 of FIG. 7, the seizure timing selector 220 detects bio-signals having a seizure probability of 99.9% or higher among 22 seizure probabilities output from the common seizure Al model. Here, n may represent a time detected as a seizure or inter-seizure in a common seizure Al model.

Next, in steps S706 and S708 of FIG. 7, the seizure timing selector 220 checks information whether the user has a seizure at the 1st, 7th, 8th, 9th, 10th, 14th, 17th, 18th, 19th, and 20th time points with a seizure probability of 99.9% or higher. At this time, seizures were identified from information regarding whether the user had a seizure at the 1st, 8th, 10th, 14th, 17th, 18th, 19th, and 20th time points.

In step S710 of FIG. 7, the seizure timing selector 220 selects the bio-signals at the time points when seizures were identified from information regarding whether the user had a seizure, as seizure signal candidates. The bio-signals at the 1st, 8th, 10th, 14th, 17th, 18th, 19th, and 20th time points may be selected as seizure signal candidates.

Next, in steps S712 and S716 of FIG. 7, the seizure timing selector 220 checks seizure types of the seizure signal candidates. The seizure timing selector 220 may identify the seizure type of each seizure signal candidate through the similarity between each seizure signal candidate and the seizure template or the similarity between the seizure signal candidates.

As a result of identifying the seizure types of the seizure signal candidates, the seizure types of the seizure signal candidates at the 1st, 8th, 10th, 17th, 18th, and 20th time points were identified to be type A, and the seizure types of the seizure signal candidates at the 14th and 19th time points were identified to be type B. As steps S718, S720, and S722 of FIG. 7, the seizure timing selector 220 may sequentially store the seizure signal candidates at the 1st, 8th, 10th, 17th, and 18th time points in the seizure signal candidate group of type A, and may sequentially store the seizure signal candidates at the 14th and 19th time points in the seizure signal candidate group of type B.

Next, in steps S724 and S726 of FIG. 7, the seizure timing selector 220 determines a seizure signal candidate group of type A in which five or more seizure signal candidates of the same seizure type are observed as a A-type seizure template, and stores the seizure signal candidate group of type A as A-type seizure template.

In step S714 of FIG. 7, since the A-type seizure template already exists, the seizure timing selector 220 may add the 20th seizure signal candidate to the A-type seizure template.

Meanwhile, since p seizure signal candidates of type B have not been observed, the seizure signal candidates of type B are not used as learning data until p or more seizure signal candidates of the type B are observed.

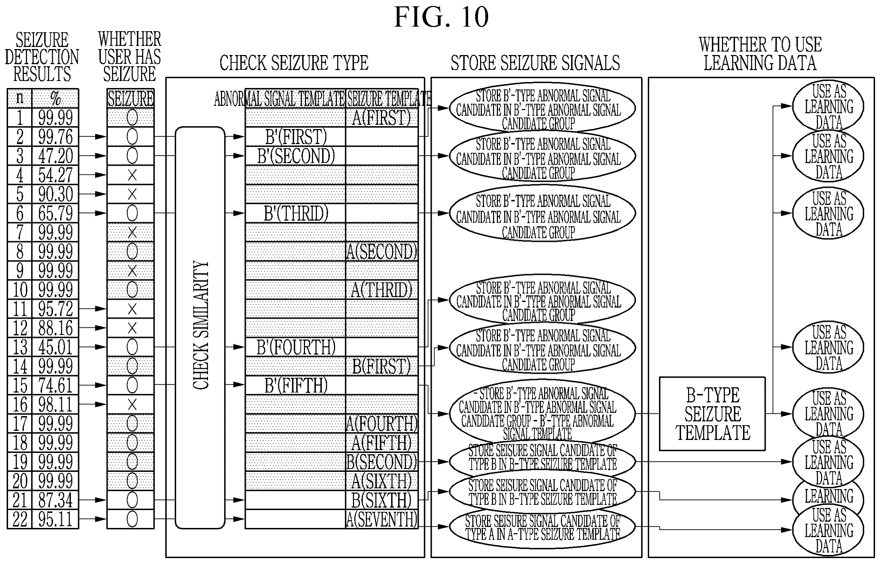

FIG. 10 is a diagram illustrating an example of generating a seizure template when the seizure probability is less than a threshold in the seizure detection and prediction results of the common seizure Al model.

Referring to FIGS. 8 and 10, the seizure timing selector 220 detects bio-signals having a seizure probability of less than 99.9% among 22 seizure probabilities output from the common seizure Al model. The seizure timing selector 220 may detect bio-signals at the 2nd, 3rd, 4th, 5th, 6th, 11th, 12th, 13th, 15th, 16th, 21st, and 22nd time points.

Next, in steps S802 and S804 of FIG. 8, the seizure timing selector 220 check information whether the user has a seizure at the 2nd, 3rd, 4th, 5th, 6th, 11th, 12th, 13th, 15th, 16th, 21st, and 22nd time points having a seizure probability of less than 99.9%. At this time, seizures were identified from information regarding whether the user had a seizure at the 2nd, 3rd, 6th, 13th, 15th, 21st, and 22nd time points.

In step S806 of FIG. 8, the seizure timing selector 220 selects the bio-signals at the time points when seizures were identified from information regarding whether the user had a seizure, as abnormal signal candidates. The bio-signals at the 2nd, 3rd, 6th, 13th, 15th, 21st, and 22nd time points may be selected as abnormal signal candidates.

Next, for steps S808 and S812 of FIG. 8, the seizure timing selector 220 checks seizure types of the abnormal signal candidates. The seizure timing selector 220 may identify the seizure type of each abnormal signal candidate through the similarity between each abnormal signal candidate and the seizure template or the similarity between the abnormal signal candidates.

As a result of identifying the seizure types of the abnormal signal candidates, the seizure types of the abnormal signal candidates at the 2nd, 3rd, 6th, 13th, and 15th time points were identified to be type B'. As steps S814, S816, and S818 of Fig. 8, the seizure timing selector 220 may sequentially store the abnormal signal candidates of the 2nd, 3rd, 6th, 13th, and 15th time points in the abnormal signal candidate group of type B'.

Next, in steps S820, S822, and S824 of FIG. 8, the seizure timing selector 220 may store an abnormal signal candidate group of type B' in which q (e.g., 5) or more abnormal signal candidates of the same seizure type B' are observed as a B'-type abnormal template, and transmit the B'-type abnormal template to a plurality of expert terminals.

As a step S826 of FIG. 8, the seizure timing selector 220 may change the B'-type abnormal template into a B-type seizure template based on the analysis results of the B'-type abnormal template from the plurality of expert terminals.

In this way, after the B-type seizure template is generated, if the seizure type of the seizure signal candidate at the 19th time point is identified to be type B, the seizure signal candidate at the 19th time point may be stored in the B-type seizure template.

In addition, if a seizure signal candidate of the same seizure type was generated before the abnormal signal template is changed to the B-type seizure template, such as a seizure signal candidate at the 14th time point, the seizure timing selector 220 may check the seizure signal candidate group when the abnormal signal template is changed to the seizure template, and determine the seizure signal candidates of the seizure signal candidate group of the same seizure type as seizure signals and add it to the corresponding seizure template.

Thereafter, in steps S808 and S8810, if the seizure type of the abnormal signal candidate at the 21st time point is identified as type B, the seizure timing selector 220 may store the abnormal signal candidate at the 21st time point as a seizure signal in the B-type seizure template, and if the seizure type of the abnormal signal candidate at the 22nd time point is identified as type A, the abnormal signal candidate at the 22nd time point may be stored as a seizure signal in the A-type seizure template.

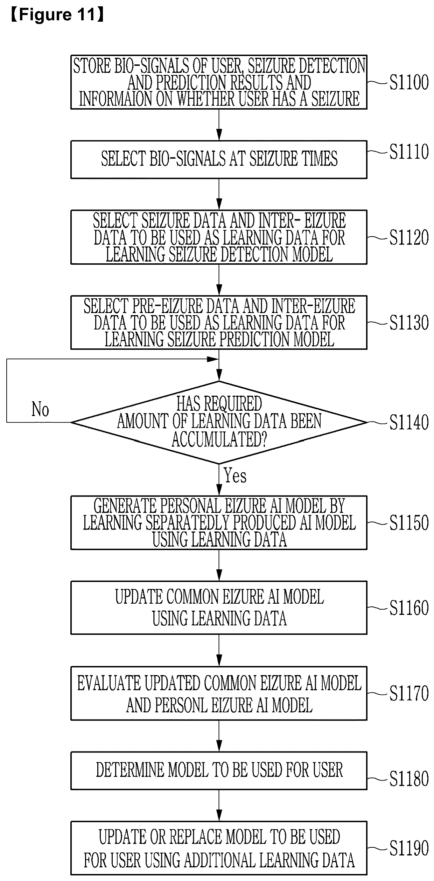

FIG. 11 is a flowchart illustrating a method for generating a personal seizure Al model in the model optimization apparatus illustrated in FIG. 6.

Referring to FIG. 11, the model optimization apparatus 200 stores the bio-signals of the user and the seizure detection and seizure prediction results for bio-signals of the user along with information on whether the user has a seizure in the memory 240, through the bio-signal accumulator 210 (S1100).

The model optimization apparatus 200 selects bio-signals at the seizure time by using the data of the user stored in the memory 240 through the seizure time selector 220 (S1110).

The model optimization apparatus 200 selects seizure data and inter-seizure data to be used as learning data for learning the seizure detection model by using the bio-signals at the seizure time through the personal model generator 231 (S1120). The seizure timing selector 220 may select bio-signals at the seizure point using the method described with reference to FIGS. 9 and 10, and the personal model generator 231 may select bio-signals at the seizure time as seizure data and select bio-signals at times other than the seizure time as inter-seizure data.

The model optimization apparatus 200 may select pre-seizure data and inter-seizure data to be used as learning data for learning the seizure prediction model by using bio-signals at the seizure time through the personal model generator 231 (S1130). The personal model generator 231 may set a time point within several minutes or several hours before the seizure time in the bio-signals to which the seizure time is applied, as a predicted time, selects the bio-signals at the predicted time as pre-seizure data for learning the seizure prediction model, and may select bio-signals at times other than the predicted time and the seizure time as inter-seizure data for learning the seizure prediction model.

When the model optimization apparatus 200 accumulates the amount of learning data required for generating a personal seizure Al model (S1140), the personal model generator 231 generates the personal seizure Al model by learning a separately produced Al model using the learning data (S1150). The model optimization apparatus 200 may learn a seizure detection model of a separately produced Al model using seizure data and pre-seizure data, and may generate the personal seizure Al model by learning a prediction detection model of a separately produced Al model using pre-seizure data and inter-seizure data.

In addition, the model optimization apparatus 200 updates the common seizure Al model using learning data through the personal model generator 231 (S1160). At this time, some of the learning data is used as evaluation data, and the evaluation data is not used to learn the model. The ratio of learning data and evaluation data may be set to 9:1 or 8:2 among the entire learning data.

The model optimization apparatus 200 evaluates the updated common seizure Al model 300 and the personal seizure Al model using evaluation data through the personal model generator 231 (S1170), and determines the model to be used for the user based on the evaluation results (S1180). The model optimization apparatus 200 may replace the model to be used for the user from the common seizure Al model to the personal seizure Al model if the performance of the personal seizure Al model is better than the common seizure Al model 300 by more than a set performance increase. Meanwhile, if the performance of the personal seizure Al model is not better than the common seizure Al model 300 by more than the set performance increase, the model optimization apparatus 200 may use the updated common seizure Al model 300 as the model to be used for the user.

Thereafter, if seizure data of the user with an interval longer than a certain period of time is additionally selected and then learning data of the user is additionally accumulated, the model optimization apparatus 200 may update or replace the model to be used for the user by using the additional learning data through the personal model generator 231 (S1190).

For example, it is assumed that the performance of the personal seizure Al model is better than that of the common seizure Al model 300 by more than a set performance increase, and thus the personal seizure Al model is determined to be the model to be used for the user. Thereafter, as learning data is additionally accumulated, the model optimization apparatus 200 updates the personal seizure Al model using the learning data excluding the evaluation data. Next, the model optimization apparatus 200 evaluates the performance of the updated personal seizure Al model and the personal seizure Al model before the update using the evaluation data, and determine the updated personal seizure Al model or the personal seizure Al model before the update as the model to be used for the user based on evaluation results.

As another example, it is assumed that the updated common seizure Al model 300 is better than the individual seizure Al model by more than a set performance increase, and thus the updated common seizure Al model 300 is determined to be the model to be used for the user. Thereafter, as learning data is additionally accumulated, the model optimization apparatus 200 updates the personal seizure Al model and the updated common seizure Al model, respectively, using the learning data excluding the evaluation data. Next, the model optimization apparatus 200 evaluates the performance of the updated common seizure Al model and the updated personal seizure Al model using the evaluation data, and may use the updated personal seizure Al model as a model to be used for the user or the updated common seizure Al model as the model to be used for the user, based on the performance results.

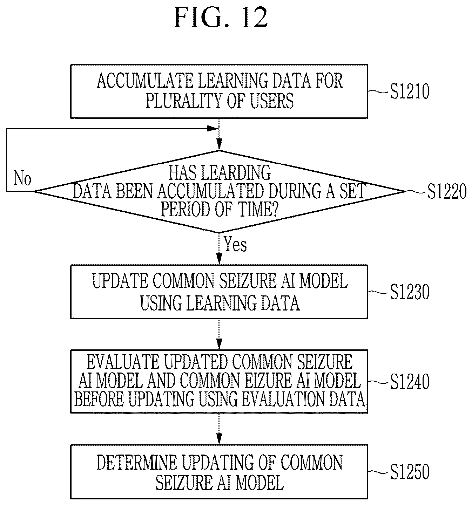

FIG. 12 is a flowchart illustrating a method for optimizing the common seizure Al model in the model optimization apparatus illustrated in FIG. 6.

Referring to Fig. 12, the model optimization apparatus 200 accumulates learning data for a plurality of users for optimization of the common seizure Al model through the bio-signal accumulator 210 and a seizure time selector 220 (S1210).

When learning data for the plurality of users is accumulated during a set period of time (S1220), the model optimization apparatus 200 learns the seizure detection model and seizure prediction model of the common seizure Al model using the learning data excluding the evaluation data through the common seizure Al model optimizer 233, thereby updating the common seizure Al model (S1230).

The model optimization apparatus 200 may evaluate the updated common seizure Al model and the common seizure Al model before update using evaluation data through the common seizure Al model optimizer 233 (S1240), and determine an update of the common seizure Al model based on the evaluation result (S1250). The model optimization apparatus 200 may use the updated common seizure Al model if the performance of the updated common seizure Al model is better than the common seizure Al model before the update by more than a set performance increase.

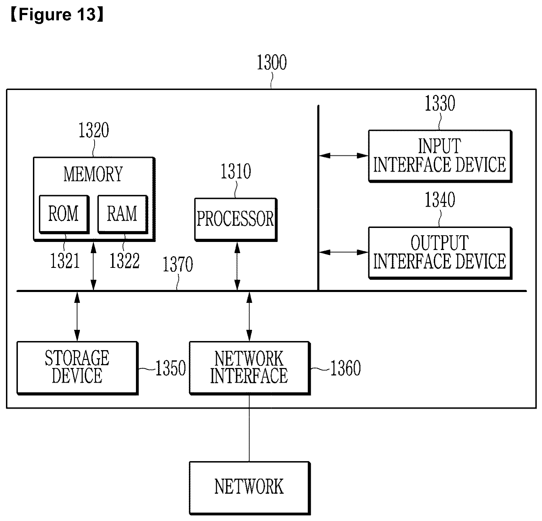

FIG. 13 is a diagram illustrating a seizure detection and prediction apparatus according to another embodiment.

Referring to FIG. 13, the seizure detection and prediction apparatus 1300 may represent a computing device in which the seizure detection and prediction method including the learning method described is implemented.

The seizure detection and prediction apparatus 1300 may include at least one of processor 1310, a memory 1320, an input interface device 1330, an output interface device 1340, a storage device 1350, and a network interface device 1360. Each of the components may be connected by a common bus 1370 to communicate with each other. In addition, each of the components may be connected through an individual interface or a separate bus centering on the processor 1310 instead of the common bus 1370.

The processor 1310 may be implemented as various types such as an application processor (AP), a central processing unit (CPU), a graphics processing unit (GPU), etc., and may be any semiconductor device that executes a command stored in the memory 1320 or the storage device 1350. The processor 210 may execute program commands stored in at least one of the memory 1320 and the storage device 1350. The processor 1310 may store program commands for implementing at least some functions of the learning unit 110 and the seizure detector and predictor 130 in the memory 1320, and control to perform the operation described with reference to FIGS. 1 to 12.

The memory 1320 and storage device 1350 may include various forms of volatile or non-volatile storage media. For example, the memory 1320 may include read-only memory (ROM) (1321) and random access memory (RAM) 1322. The memory 1320 may be located inside or outside the processor 1310, and the memory 1320 may be connected to the processor 1310 via various means already known. The memory 1320 or storage device 1350 may include the memory 120 illustrated in FIG. 2.

The input interface device 1330 may be configured to provide input data to the processor 1310.

The output interface device 1340 may be configured to output data from the processor 1310.

The network interface device 1360 may transmit or receive signals to or from an external device via a wired network or a wireless network.

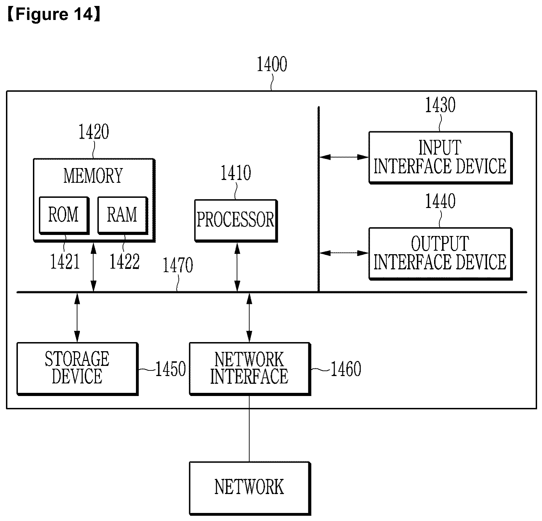

FIG. 14 is a diagram showing a model optimization apparatus according to another embodiment.

Referring to FIG. 14, the model optimization apparatus 1400 may represent a computing device in which the model optimization method described above is implemented.

The model optimization apparatus 1400 may include at least one of processor 1410, a memory 1420, an input interface device 1430, an output interface device 1440, a storage device 1450, and a network interface device 1460. Each component is connected to a common bus 1470 and can communicate with each other. In addition, each of the components may be connected through an individual interface or a separate bus centering on the processor 1410 instead of the common bus 1470.

The processor 1410 may be implemented as various types such as an AP, a CPU, a GPU, etc., and may be any semiconductor device that executes a command stored in the memory 1420 or the storage device 1450. The processor 1410 may execute program commands stored in at least one of the memory 1420 and the storage device 1450. The processor 1410 may store program commands for implementing at least some functions of the bio-signal accumulator 210, the seizure timing selector 220, and the Al model optimizer 230 in the memory 1320, and control the operations described based on FIGS. 1 to 12 to be performed.

The memory 1420 and storage device 1450 may include various forms of volatile or non-volatile storage media. For example, the memory 1420 may include ROM 1421 and RAM 1422. The memory 1420 may be located inside or outside the processor 1410, and the memory 1420 may be connected to the processor 1410 via various means already known. The memory 1420 or storage device 1350 may include the memory 240 illustrated in FIG. 2.

The input interface device 1430 may be configured to provide input data to the processor 1410.

The output interface device 1440 may be configured to output data from the processor 1410.

The network interface device 1460 may transmit or receive signals to or from an external device via a wired network or a wireless network.

Although the embodiments of the present disclosure have been described in detail above, the scope of the present disclosure is not limited thereto, and various modifications and improvements of those skilled in the art using the basic concepts of the present disclosure defined in the following claims are also included in the present disclosure.

## Claims

1. A seizure monitoring method of a user which is executed by at least one processor, the seizure monitoring method comprising:
detecting seizure detection and prediction results from bio-signals of the user by using a common seizure Al model that compares similarity with a common seizure template composed of bio-signals collected from a plurality of users;
accumulating user data including the bio-signals of the user and seizure detection and prediction results during a first period of time;
updating the common seizure template and the common seizure Al model using the accumulated user data;
generating a personal seizure Al model using the accumulated user data;
determining a model optimized for the user among the updated common seizure Al model or personal seizure Al model; and
detecting seizure detection and prediction results from the bio-signals of the user using the model optimized for the user.

2. The seizure monitoring method of claim 1, further comprising:
updating or replacing the model optimized for the user by using user data additionally accumulated during a second period of time after the first period of time.

3. The seizure monitoring method of claim 1, wherein
the determining includes:
evaluating the updated common seizure Al model and the personal seizure Al model using evaluation data selected from the accumulated user data; and
selecting the personal seizure Al model as the optimized model for the user if a performance of the personal seizure Al model is better than the updated common seizure Al model by more than or equal to a set performance increase, based on evaluation results.

4. The seizure monitoring method of claim 1, further comprising:
selecting seizure data and pre-seizure data to be used as learning data from the user data before the updating.

5. The seizure monitoring method of claim 4, wherein
the seizure detection and prediction results include a seizure probability at each time point, and
the selecting includes:
receiving user seizure confirmation information including whether the user has an actual seizure and a seizure time from the user terminal;
identifying whether an actual seizure occurred to the user at time points having a seizure probability higher than or equal to a threshold among the bio-signals of the user through the user seizure confirmation information; and
determining seizure data by using bio-signals at time points at which an actual seizure is identified to have occurred to the user among the time points having a seizure probability higher than or equal to the threshold value for the bio-signal of the user.

6. The seizure monitoring method of claim 5, wherein
the determining the seizure data includes:
identifying a seizure type of a bio-signal at each time point having a seizure probability greater than or equal to the threshold; and
storing bio-signals of a first seizure type as the seizure data in a seizure template of the first seizure type when a set number or more of bio-signals of the first seizure type are observed among the bio-signals at time points at which the actual seizure is identified to have occurred to the user while having a seizure probability greater than or equal to the threshold.

7. The seizure monitoring method of claim 6, wherein
the determining the seizure data further includes storing the bio-signals at a time point having the first seizure type in a seizure signal candidate group of the first seizure type until the set number or more of bio-signals at a time point having the first seizure type are observed.

8. The seizure monitoring method of claim 6, wherein
the determining the seizure data further includes, if a seizure template of a seizure type identical to a bio-signal at a time point at which the actual seizure is identified to have occurred to the user while having a seizure probability greater than or equal to the threshold exists, storing the bio-signal at the time point as the seizure data in the seizure template.

9. The seizure monitoring method of claim 5, wherein
the selecting includes:
identifying whether an actual seizure occurred to the user through the user seizure confirmation information at time points having a seizure probability less than the threshold among the bio-signals of the user; and
determining the seizure data by using bio-signal at time points at which an actual seizure is identified to have occurred to the user among bio-signals at time points having a seizure probability less than the threshold.

10. The seizure monitoring method of claim 9, wherein
the determining the seizure data includes:
identifying a seizure type of a bio-signal at each time point having a seizure probability less than the threshold;
storing bio-signals of a second seizure type as an abnormal signal data in an abnormal signal template of the second seizure type, when a set number or more of bio-signals of the second seizure type are observed among the bio-signals at time points at which the actual seizure is identified to have occurred to the user while having a seizure probability less than the threshold; and
changing the abnormal signal template of the second seizure type as the seizure data to a seizure template of the second seizure type based on analysis results of the abnormal signal template of the second seizure type.

11. The seizure monitoring method of claim 10, wherein
the determining the seizure data further includes storing the bio-signals at a time point having the second seizure type in an abnormal signal candidate group of the second seizure type until the set number or more of bio-signals at a time point having the second seizure type are observed.

12. The seizure monitoring method of claim 10, wherein
the determining the seizure data further includes, if a seizure template of a seizure type identical to a bio-signal at a time point at which the actual seizure is identified to have occurred to the user while having a seizure probability less than the threshold exists, storing the bio-signal at the time point as the seizure data in the seizure template.

13. The seizure monitoring method of claim 4, wherein
the selecting includes storing the seizure data selected seizure data from the user data in a seizure template for each seizure type, and
the updating includes storing the seizure template for each seizure type in the common seizure template.

14. The seizure monitoring method of claim 1, further comprising:
accumulating data of the plurality of users including bio-signals of plurality of users and seizure detection and prediction results during a third period of time; and
optimizing the common seizure Al model by using the data of the plurality of users accumulated during the third period of time.

15. The seizure monitoring method of claim 14, wherein
the optimizing includes:
evaluating the common seizure Al model updated using the data of the plurality of users and the common seizure Al model before the update using evaluation data selected from the data of the plurality of users accumulated during the third period of time; and
determining one of the updated common seizure Al model and the common seizure Al model before the update as the optimized common seizure Al model based on evaluation results using the data of the plurality of users.

16. The seizure monitoring method of claim 1, wherein
the detecting includes:
preprocessing the bio-signals of the user; and
inputting the preprocessed data into the common seizure Al model.

17. The seizure monitoring method of claim 16, wherein
the preprocessing includes extracting feature data to be used as input for the common seizure Al model.

18. The seizure monitoring method of claim 1, wherein
the bio-signals of the user includes a bio-signal of a new user who does not have the acquired bio-signal or a bio-signal of a new seizure type that is not included in a seizure template of an existing user whose bio-signal has already been input into the common seizure Al model.

19. The seizure monitoring method of claim 1, further comprising:
detecting seizure detection and prediction results from the bio-signals of the user using the model optimized for the user.

20. A seizure detection and prediction apparatus for seizure monitoring, the seizure detection and prediction apparatus comprising:
a memory configured to store acquired bio-signals;
a learning unit configured to learn a plurality of candidate seizure Al models using learning data selected from the acquired bio-signals, determine one of the plurality of candidate seizure Al models as a common seizure Al model to be used for seizure detection and prediction using evaluation data selected from the acquired bio-signals, and store the common seizure Al model in the memory; and
a seizure detector and predictor configured to load the common seizure Al model stored in the memory and output seizure detection and prediction results from the bio-signals of the user using the common seizure Al model.

21. The seizure detection and prediction apparatus of claim 20, wherein
the seizure detector and predictor includes:
a bio-signal preprocessor configured to preprocess the bio-signals of the user and extract feature data;
a result calculator configured to generate seizure detection and seizure prediction results by using a seizure probability and a pre-seizure probability output from the common seizure Al model that received the feature data as input; and
output unit configured to output the seizure detection and seizure prediction results.

22. The seizure detection and prediction apparatus of claim 21, wherein
the common seizure Al model includes, at least one of a seizure detection model configured to output the seizure probability from the feature data and a seizure prediction model configured to output pre-seizure probability from the feature data.

23. The seizure detection and prediction apparatus of claim 20, wherein
the common seizure Al model stored in the memory is configured to update or replace using the seizure detection and seizure prediction results of the plurality of users accumulated during a set period of time.

24. A model optimization apparatus that optimizes a seizure detection and prediction model for seizure detection, the model optimization apparatus comprising:
a bio-signal accumulator configured to accumulate user data including seizure detection and prediction results detected from bio-signals of a user during a first period of time by using a common seizure Al model learned using learning data selected from acquired bio-signals;
a seizure timing selector configured to select bio-signals at seizure times from the user data accumulated during the first period of time; and
a personal model generator configured to select learning data and evaluation data from user data using the bio-signals at the seizure times, update the common seizure Al model using the learning data, generate a personal seizure Al model using the learning data, and determine a model optimized for the user among the updated common seizure Al model or the personal seizure Al model.

25. The model optimization apparatus of claim 24, wherein
the personal model generator is configured to evaluate the updated common seizure Al model and the personal seizure Al model using the evaluation data, and select the personal seizure Al model as the model optimized for the user if a performance of the personal seizure Al model is better than a performance of the updated common seizure Al model based on evaluation results.

26. The model optimization apparatus of claim 24, wherein
the common seizure Al model optimizer is configured to select learning data and evaluation data from the data of a plurality of users by using bio-signals at seizure times of the plurality of users selected from the user data of the plurality of users accumulated during a second period of time, and optimize the common seizure Al model by using the learning data selected from the data of the plurality of users.

27. The model optimization apparatus of claim 26, wherein
the common seizure Al model optimizer is configured to update the common seizure Al model using learning data selected from the data of the plurality of users, and evaluate the updated common seizure Al model and the common seizure Al model before the update using evaluation data selected from the data of the plurality of users to determine an optimized common seizure Al model.

28. The model optimization apparatus of claim 24, wherein
the seizure detection and prediction results include a seizure probability at each time point,
the bio-signal accumulator is configured to accumulate user seizure confirmation information including whether the user has an actual seizure and a seizure time, received from the user terminal, as the user data, and
the seizure timing selector is configured to determine seizure data by using bio-signals at time points at which an actual seizure is identified to have occurred to the user through user seizure confirmation information, among the time points having a seizure probability higher than or equal to the threshold value for the bio-signals of the user.

29. The model optimization apparatus of claim 28, wherein
the seizure timing selector is configured to determine the seizure data by using bio-signals at time points at which an actual seizure is identified to have occurred to the user through user seizure confirmation information, among at time points having a seizure probability less than the threshold for the bio-signals of the user.

30. The model optimization apparatus of claim 24, wherein
the common seizure Al model and the personal AI seizure model include least one of a seizure detection model configured to output a seizure probability and seizure prediction model configured to output a pre-seizure probability,
the personal model generator is configured to set the bio-signals at seizure times as seizure data and set bio-signals at time points other than the seizure time as inter- seizure data, and select them as learning data for learning a seizure detection model, and
the personal model generator is configured to set bio-signals at a predetermined predicted time point prior to the seizure time as pre-seizure data and set bio-signals at time points other than the seizure time and the predicted time point as inter-seizure data, and select them as learning data for learning the seizure prediction model.
